# EUROPEAN PATENT APPLICATION

(11) **EP 1 228 752 A2**
(43) Date of publication of application: **07.08.2002**
(21) Application number: 02002311.5
(22) Date of filing: 31.01.2002
(51) Int. Cl.: A61K 7/48

(54) **Cosmetic compositions containing natural yeast cells**

(30) Priority: 02.02.2001 IT MI010207
(71) Applicant: Istituto Profilattico Italiano Torino-I.P.I.T. S.r.l., 10040 Leini (TO) (IT)
(72) Inventor: Barbesino, Claudio, 10040 Leini (TO) (IT); Ferrero, Chiara, 10040 Leini (TO) (IT)
(74) Representative: Cioni, Carlo

(57) **Abstract**

Cosmetic compositions of a natural type, not comprising additives or components of synthetic origin, which are at least as efficacious as the corresponding conventional cosmetic compositions, characterized by comprising two or more separated components, one of which is yeast, preferably in the lyophilized state and in any case as the nutrient substrate of the said yeast, the other component(s) being constituted from the active principles preferably in the form of suspension or concentrated watery solution.

## Description

The present invention relates to compositions for the cure and the aesthetic treatment of the skin that contains, in addition to the customary cosmetic bases, an efficacious quantity of yeast cells and proteins deriving from the fermentation of the said yeast on determined substrates.

### Description and discussion of the state of the art

Cosmetic compositions in current use are supplied in the form of creams or salves and, in general, compositions for topical treatments are produced and sold as emulsions or pseudo-emulsions containing the active principles. This resort to emulsions is due to the need to guarantee both homogeneity of composition over time and greater efficacy of the cosmetic treatments. In order to stabilize the emulsion and avoid degradation of the same, a considerable quantity of components or additives are added which serve to maintain the cosmetic composition in the emulsion form despite the presence of other agents such as disinfectants, preservatives, bactericides, bactero-stabilizers and the like.

The presence in cosmetic compositions of the components and additives mentioned above is not desired by users of these cosmetic compositions and for this reason research has been directed towards the formulation of compositions - known as natural compositions - containing ever fewer, and smaller quantities, of the said additives or components.

However, the search for natural-origin components like surfactants, not to speak of bactero-static, preserving, disinfecting, antioxidant additives or components, etc. (always of natural origin) has not yielded the hoped-for results.

### Summary of the invention

It is thus a primary aim of the present invention to produce cosmetic compositions of a natural type, i.e. not comprising additives or components of synthetic origin, that are at least as efficacious as the corresponding conventional cosmetic compositions, and that are not subject to the attack by bacteria or other chemical agents.

A secondary aim of the present invention is to use a composition as defined below for the preparation of a cosmetic and/or therapeutic composition.

The principle on which the present invention is based is the discovery that a cosmetic composition in the form of an emulsion could be obtained by using yeast which, added to a solution of the cosmetic active principle and in the presence of substrate, causes gas to be released which, diffusing into the solid and liquid phases, gives rise to an emulsion or foam which can be used directly without needing to add any conserving or disinfecting agents.

### Detailed description of the invention

The aims of the present invention are jointly achieved by the aforementioned procedure and composition according to which the cosmetic composition comprises two or more separate components, one of which consists of yeast, preferably in the lyophilized state or otherwise as a solid fermentation substrate, the other component(s) being constituted by the active principles, preferably in concentrated aqueous solution or suspension.

Typically the components are stored in separate containers and only mixed just before use. The substrate nutrients can also be kept separated from the yeast in order to prevent premature reaction between yeast and substrate.

The quantity of yeast used to prepare the composition according to the present invention ranges from 2% to 70% of the total weight of the composition; greater amounts of yeast can be used, but do not improve the results. Any type of yeast that reacts with substrate to produce a gas can be used; those varieties of yeast commonly used in the food industry, such as Brewer's Yeast (Saccaromices Cerevisiae), are preferred.

Any conventional material which reacts with the chosen yeast to release an innocuous gas (such as CO₂ for example) and produce a foam by mechanical means, can be used as nutrient substrate. Typically, the nutrient substrates used are sugars and/or starches; the products obtained comprise emulsions of proteins, vitamins, enzymes, fibers, glucosides and lipids present in the initial substrate, which are emulsified, modified and integrated by the metabolic activity of the yeast cells.

The use, in cosmetic preparations, of yeast and its metabolic activity, introduces a series of advantages over conventional cosmetic compositions.

The labor cost of the cosmetic finished product is practically nil in the process according to the present invention, the only task left to the user is to produce the final composition. On the other hand, the preparation of analogous commercial cosmetic compositions involves the use of the cosmetic industry's own equipment such as emulsifying plant, mixers etc.

The cost of materials for obtaining the finished product is far smaller in the case of the present invention, being limited to the essential components, the active principles, yeast and the nutrient substrate.

Another advantage lies in the fact that, as pointed out above, the cosmetic composition is prepared substantially at the moment of use and therefore does not need antioxidant, disinfecting, preserving, stabilizing etc. additives to be present.

A further characteristic of the invention derives from the synergy effect between the active principle and substrate according to the present invention compared to the compositions of the known technology. It has been found that, for the same quantity of active principle, the compositions that exploit the emulsifying action of yeast and the nutrient substrate, have given considerably better results. These synergy effects between substrate and the active principle have been found with compositions comprising most of the active principles.

The cosmetic products that can be prepared with the procedure according to the invention are, as stated above, those that act on the skin, such as salves and creams, for example. Other beauty products which can be produced in the form envisaged by the present invention are the compresses, such as the compresses for hair in order to prevent the formation of dandruff or for treating greasy or dry hair; masks, called face/neck masks, for the treatment of dry or greasy skins, or masks simply for hydrating skins; masks for the body, also those with hydrating function. They can moreover be used as foam for the treatment of the hands or the production of specific products like adjuvant compresses in the treatment of cellulite.

The invention will now be described in more detail with the aid of the examples that follow which show, for comparison, some prescriptions for the production of the compositions according to the invention and prescriptions for equivalent commercially available cosmetic products. It is obvious that the list of compositions produced according to the present invention should not be interpreted as limiting the scope of the invention.

### Preparation of the cosmetic compositions

The cosmetic compositions containing yeast according to the invention, have been prepared by simple manually-executed mixing. By way of example, the preparation of a product used for a mask for impure skins has the following stages, all manual.

### A) PREPARATION OF A PRODUCT CONTAINING YEAST ACCORDING TO THE INVENTION

| MODEL A | |
|---|---|
| | RAW MATERIAL |
| PHASE A | STARCHES/THICKENING AGENTS |
| | SACCHAROSE |
| PHASE B | LYOPHILIZED YEAST |
| PHASE C | AQUEOUS EXTRACT |

### PROCEDURE A:

1) Pour the components of phase A and phase B into phase C
2) Mix until a homogenous mixture is obtained.
3) Leave aside for some minutes for the cosmetic properties of the preparation to develop and the composition is ready for application.

| MODEL B | |
|---|---|
| | RAW MATERIAL |
| PHASE A | STARCH/THICKENING AGENTS |
| | SACCHAROSE |
| | DRY EXTRACT |
| PHASE B | LYOPHILIZED YEAST |
| PHASE C | WATER |

### PROCEDURE B:

1) Pour the components of phase A and phase B into phase C
2) Mix until a homogenous mixture is obtained.
3) Leave aside for some minutes for the cosmetic properties of the preparation to develop and the composition is ready for application.

| MODEL C | |
|---|---|
| | RAW MATERIAL |
| PHASE A | STARCH/THICKENING AGENTS |
| | SACCHAROSE |
| | WATER |
| PHASE B | YEAST LYOPHILIZED |
| | DRY EXTRACT |

### PROCEDURE C:

1) Pour the components of B into phase A
2) Mix until a homogenous mixture is obtained.
3) Leave aside for some minutes for the cosmetic properties of the preparation to develop and the composition is ready for application.

| MODEL D | |
|---|---|
| | RAW MATERIAL |
| PHASE A | STARCH/THICKENING AGENTS |
| | SACCHAROSE |
| | YEAST LYOPHILIZED |
| PHASE B | AQUEOUS EXTRACT |

### PROCEDURE D:

1) Pour the components of phase A into phase B
2) Mix until a homogenous mixture is obtained.
3) Leave aside for some minutes for the cosmetic properties of the preparation to develop and the composition is ready for the application.

| MODEL E | |
|---|---|
| | RAW MATERIAL |
| PHASE A | STARCH/THICKENING AGENTS |
| | SACCHAROSE |
| | DRY EXTRACT |
| PHASE B | YEAST LYOPHILIZED |
| PHASE C | WATER |

### PROCEDURE E:

1) Pour the components of phase A and phase B into phase C
2) Mix until a homogenous mixture is obtained.
3) Leave aside for some minutes for the cosmetic properties of the preparation to develop and the composition is ready for application.

| MODEL F | |
|---|---|
| | RAW MATERIAL |
| PHASE A | STARCH/THICKENING AGENTS |
| | SACCHAROSE |
| | DRY EXTRACT |
| | YEASTLYOPHILIZED |
| PHASE B | WATER |

### PROCEDURE F:

1) Pour the components of phase A into phase B
2) Mix until a homogenous mixture is obtained.
3) Leave aside for some minutes for the cosmetic properties of the preparation to develop and the composition is ready for application.

| MODEL G | |
|---|---|
| | RAW MATERIAL |
| PHASE A | STARCH/THICKENING AGENTS |
| | SACCHAROSE |
| | AQUEOUS EXTRACT |
| PHASE B | YEAST LYOPHILIZED |

### PHASE A OF MODEL G MUST BE STERILIZED FOR LONG CONSERVATION.

### PROCEDURE G:

1) Pour the components of phase B into phase A
2) Mix until a homogenous mixture is obtained.
3) Leave aside for some minutes for the cosmetic properties of the preparation to develop and the composition is ready for application.

**N.B.** IN EVERY MODEL A SUBSTANCE SUCH AS AN ORGANOLEPTIC CORRECTIVE CAN BE ADDED IN ONE OF THE PHASES.

### Preparation of conventional cosmetic compositions.

The conventional cosmetic compositions were prepared based on prescriptions well known to technicians in the field; as an example the composition analogous to the one mentioned above and reported here contains the ingredients listed in table B)

### B) EXAMPLE OF WORKSHEET FOR A NORMAL COSMETIC PRODUCT

| | Raw material | function | qty/kg |
|---|---|---|---|
| PHASE A | WATER | | 770 |
| | PEMULEN TR-2 | THICKENING AGENTS | 2 |
| PHASE B | GLUCATE DO | MOISTURIZER | 1 |
| | ISOPROPYLPALMITATE | BINDER | 90 |
| | SILICONE OIL | EMOLLIENT | 5 |
| | RHODISTEROL | FUNCTIONALIZER | 50 |
| PHASE C | GLYCEROL | MOISTURIZER | 30 |
| | PARABENI IN FENOSS. | PRESERVATIVE | 5 |
| PHASE D | WATER | | 30 |
| | KEMIPUR 100 | PRESERVATIVE | 3 |
| | BISODIUM EDTA | CHELATING AGENT | 1 |
| | ACEPUR K | PRESERVATIVE | 0,5 |
| PHASE E | WATER | | 10 |
| | TRIETHANOLAMINE | BUFFER | 2 |
| PHASE F | SALCARE SC 91 | BINDER/EMOLLIENT | 10 |
| PHASE G | LIPOMOIST 2013 | RHEOLOGICAL/ MOISTURIZER ADDITIVE | 30 |
| PHASE H | PERFUME | | 7,5 |

The composition is prepared in the following way:
1) Water (740g / kg) is placed in a turbo-emulsifier (TBM) taking evaporation into account, heated to 80°C and the Pemulen TR2 is sprinkled in under agitation. The agitation is continued for another 20 minutes under vacuum.
2) The components of phase B are placed in the reactor and heated to 65-70°C. When the mass is melted, add component B' and maintain under the action of the TBM to homogenize.
3) Composition B-B' is poured slowly into phase A, maintaining the TBM in function.
4) Add phase C pre-mixed; it is maintained under TBM for approximately 1 minute, then begin slow cooling under agitation.
5) Phase D is prepared apart by adding components one at a time in sequence, under agitation, until a clear solution is obtained, then added into the mixer at 45°C.
6) Neutralize by adding phase E premixed while agitating. The cooling is resumed, gradually raising the vacuum.
7) At 40° C add phase F under the action of the TBM.
8) Add the components of phase G under agitation; agitate well.
9) Add the components of phase H under agitation; set the TBM in action for some seconds in order to make the product quite uniform. Resume cooling to ambient temperature under agitation and maximum pressure.
10) Decant the product into the final containers.

In the examples that follow, only the ingredients of the cosmetic compositions to be inserted into the cosmetic products are reported and not the insertion processes.

### B) COMPARISON BETWEEN THE INGREDIENTS OF A CONVENTIONAL MASK FOR IMPURE SKINS AND THOSE OF A MASK USING YEAST

| WATER | AQUEOUS EXTRACT OF PINEAPPLE |
|---|---|
| GLYCOL EXTRACT OF PINEAPPLE | LYOPHILIZED YEAST(Saccharomices cerevisiae) |
| HYDROXYETHYLCELLULOSE | STARCH |
| POLYVINYLPIRROLIDONE | SACCHAROSE |
| MONOCOCCOATO GLYCERYL (7) OE | |
| HECTORITE | |
| COPODIOL (75) OE (30) OP (75) OE | |
| CALCIUM CARBONATE | |
| TALC | |
| CACCOILAMIDE PROPYLBETAINE | |
| PERFUME | |
| IMOIDAZOILIDINILUREA | |
| CHLOROMETHYLISOTHIAZOLINONE | |
| + METHYLOXYTHIAZOLINONE | |
| PARAOXYBENZOATES IN PHENOXYETHANOL | |

### D) COMPARISON BETWEEN THE INGREDIENTS OF A HAND TREATMENT AND A TREATMENT USING YEAST.

| WATER | AQUEOUS EXTRACT of CALENDULA |
|---|---|
| EXTRACT OF CALENDULA | LYOPHILIZED YEAST (Saccharomices cerevisiae) |
| METHYLGLUCOSE (20) OE SESQUISTEARATE | STARCH |
| CETILIC ALCOHOL | SACCHAROSE |
| JOJOBA FLUID WAX | |
| KARITE' BUTTER | |
| AVOCADO OIL | |
| INSAPONIFICABLE OLIVE OIL | |
| DIMETHYLPOLYSILOXANES | |
| TOCOFEROL | |
| HYDROXYETHYLCELLULO SE | |
| GLYCEROL | |
| COLLAGEN EXTRACT | |
| METHYLGLUCOSOSESQUISTEARATE | |
| PERFUME | |
| IMIDAZOLIDINYLUREA | |
| CHLOROMETHYLISOTHIAZOLINONE + METHYLISOTIAZOLINONE | |

### E) COMPARISON BETWEEN THE INGREDIENTS OF A COMPRESS FOR HAIR (SOFTENING MASK) AND A COMPRESS USING YEAST.

| | |
|---|---|
| WATER | AQUEOUS EXTRACT OF CHAMOMILE |
| EXTRACT OF CHAMOMILE | LYOPHILIZED YEAST (Saccharomices cerevisiae) |
| INSAPONIFICABLE OIL | STARCH |
| CETILIC ALCOHOL | SACCHAROSE |
| TRIDECIL - O - HYDROXYBENZOATE | |
| PROPYLENEGLYCOL | |
| CETYLSTEARYLDECAETHOXYAMMONIUM PHOSPHATE | |
| DIMETHYLPOLYSILOXANES | |
| LANOLIN (75) OE | |
| CITRIC ACID | |
| HYDROXYETHYL CELLULOSE TRIMETHYLAMMONIUM CHLORIDE | |
| STEARYLTRIMETHYLAMMONIUM POLYPEPTIDE OF COLLAGEN | |
| PERFUME | |
| IMIDAZOLIDINYLUREA | |
| CHLOROMETHYLISOTHIAZOLINONE + METHYLISOTHIAZOLINONE | |

### F) COMPARISON BETWEEN THE INGREDIENTS OF AN ADJUVANT TREATMENT AGAINST THE UNESTHETIC EFFECTS OF CELLULITE AND A TREATMENT USING YEAST.

| | |
|---|---|
| WATER | WATER |
| EXTRACT OF RHODISTEROL (Alga gelidium cartilagineum) | EXTRACT OF RHODISTEROL (Alga gelidium cartilagineum) |
| GLYCEROL | LIOFILOZZATO YEAST (Saccharomices cerevisiae) |
| METHYLGLUCOSE DIOLEATE | STARCH |
| PALMITATE OF ISOPROPILE | SACCHAROSE |
| D - GLUCOSE | |
| CARRAGENATES | |
| GLUCURONIC ACID | |
| LINEAR DEMITHYLPOLYSILOXANE | |
| PARAOXYBENZOATES IN PHENOXYETHANOL | |
| ACRYLATES/C10 - 30 ALKYL ACRYLATE CROSSPOLYMER | |
| VASELINE OIL | |
| PPG - 1 TRIDECETH - 6 | |
| TRIETHANOLAMINE | |
| PERFUME | |
| TOCOFEROLS | |
| CITRIC ACID | |
| EDTA DISODIUM SALT | |
| IMIDAZOLIDINYLUREA | |
| METHYLCLOROISOTIAZOLINONE + METHYLISOTIAZOLINONE | |

### REPORT OF TESTS OF PROTOTYPE PRODUCT

| PRODUCT : MASK FOR DRY HAIR (*) | | | |
|---|---|---|---|
| Subject | age | sex | Result |
| 1 | 35 | F | Optimum |
| 2 | 27 | F | Optimum |
| 3 | 31 | M | Good |
| 4 | 19 | F | Good |
| 5 | 44 | F | Optimum |
| 6 | 48 | F | Optimum |
| 7 | 58 | F | Good |
| 8 | 32 | F | Optimum |
| 9 | 39 | M | Optimum |
| 10 | 20 | F | Optimum |

| PRODUCT: MASK FOR FACE (*) | | | |
|---|---|---|---|
| subject | age | sex | Result |
| 1 | 35 | F | Optimum |
| 2 | 27 | F | Optimum |
| 3 | 31 | M | Good |
| 4 | 19 | F | Optimum |
| 5 | 44 | F | Good |
| 6 | 48 | F | Optimum |
| 7 | 58 | F | Good |
| 8 | 32 | F | Good |
| 9 | 39 | M | Optimum |
| 10 | 20 | F | Optimum |

| | | | |
|---|---|---|---|
| (*) the masks were applied three times and for periods of 15 minutes. | | | |

## Claims

1. Cosmetic compositions of a natural type, not comprising additives or components of synthetic origin, which are at least as efficacious as the corresponding conventional cosmetic compositions, **characterized by** comprising two or more separate components, one of which is yeast, preferably in the lyophilized state or in any case as the nutrient substrate of said yeast, the other component(s) comprising the active principles preferably in the form of concentrated aqueous solution or suspension.

2. Cosmetic compositions of a natural type, not comprising additives or components of synthetic origin, which are at least as efficacious as the corresponding conventional cosmetic compositions, **characterized by** the components being contained in separate containers and being mixed for use.

3. Cosmetic compositions of a natural type, not comprising additives or components of synthetic origin, which are at least as efficacious as the corresponding conventional cosmetic compositions, **characterized by** the yeast used being chosen from those varieties of yeast commonly used in the food industry, such as Brewer's Yeast (Saccaromices Cerevisiae).

4. Cosmetic compositions of a natural type, not comprising additives or components of synthetic origin, which are at least as efficacious as the corresponding conventional cosmetic compositions, **characterized by** the nutrient substrate being any conventional material able to react with the chosen yeast and release an innocuous gas, such as CO₂ for example, and to produce protein, also innocuous.

5. Cosmetic compositions of a natural type, not comprising additives or components of synthetic origin, which are at least as efficacious as the corresponding conventional cosmetic compositions, **characterized by** the nutrient substrates used being sugars or starches.

6. Cosmetic compositions of a natural type, not comprising additives or components of synthetic origin, which are at least as efficacious as the corresponding conventional cosmetic compositions, **characterized by** the content of yeast ranging from 2% to 70% of the total cosmetic composition.
